# EUROPEAN PATENT APPLICATION

(11) **EP 4 174 189 A1**
(43) Date of publication of application: **03.05.2023**
(21) Application number: 21205250.0
(22) Date of filing: 28.10.2021
(51) Int. Cl.: C12Q 1/6869

(54) **ENZYME DIRECTED BIOMOLECULE LABELING**

(71) Applicant: Volker, Leen, 3470 Kortenaken (BE)
(72) Inventor: Leen, Volker, Kortenaken (BE); Leen, Volker, 3470 Kortenaken (BE)

(57) **Abstract**

Methods and compounds for labeling of biomolecules are disclosed. The method comprises combining a biomolecule-specific macromolecule and a reactive macromolecule ligand to effect labeling at or near known locations on the biomolecule.

## Description

### Technical field of the invention

Embodiments herein relate generally to labeling biomolecules, for example for use in genomic analysis

### References

- Gottfried A. et al. "Sequence-specific covalent labelling of DNA", Biochemical Society Transactions, 39(2), 623-628
- Biomolecular labeling, US6,657,052 B1
- Compounds and processes for single-pot attachment of label to nucleic acid, US2006/0188927
- Proudnikov D., et al, (1996), Chemical methods of DNA and RNA fluorescent labeling, Nucleic Acids Research, , Vol. 24, 4535-4532
- Biomolecular labeling, US6,657,052 B1
- Selection of single nucleic acids based on optical signature, US2014/0011686
- Methods of specifically labeling nucleic acids using CRISPR/CAS, US 2016/0168621
- Methods and devices for single-molecule whole genome analysis US8628919
- Ferreira de Freita R. et al., "Methyltransferase Inhibitors: Competing with, or Exploiting the Bound Cofactor"

### Background of the invention

Methyltransferase enzymes (MTases) catalyze the transfer of an activated methyl group to a range of substrates; such as polynucleotides, polypeptides, sugars and small molecules. Most methyltransferases use S-adenosyl-L- methionine (AdoMet) as a cofactor to achieve this. DNA methyltransferases transfer methyl groups to nucleotides within their DNA recognition sequences (Cheng, (1995) Annu. Rev. Biophys. Biomol. Struct. 24, 293- 318). DNA methylation is an important biological mechanism that regulates gene expression in vertebrate animals including humans (Bird, (2002) Genes Dev. 16, 6-21), Goll, M. G. & Bestor, T.H. Annu. Rev. Biochem. 74, 481-514 (2005) and serves as a species self-code in bacteria to protect themselves from invading phages. The AdoMet cofactor is universal for most methylation reactions in living organisms. This biologically and chemically active compound is comprised of a positively charged sulfonium center which joins three peripheral parts: the transferable methyl group, the adenosyl moiety and the homoserine moiety. The adenosyl and amino acid moieties typically serve as anchors which are required for discrete binding and correct orientation of the methyl group in a methyltransferase enzyme. The sulfonium center is thought to activate the methyl group for its transfer onto nucleophilic targets.

The ability of methyltransferases to catalyze sequence-specific, covalent modifications of biopolymers makes them powerful tools for biotechnology. Recently, labeling strategies using designer cofactors for DNA methyltransferases have been presented (Klimasauskas and Weinhold, (2007) Trends Biotechnol. 25, 99-104). One such strategy is based on replacing the methylgroup and the homoserine moiety of the natural cofactor S-adenosyl-L-methionine (AdoMet) by an aziridinyl moiety. These analogs confer methyltransferase-directed nucleophilic opening of the aziridine ring and coupling of the whole cofactor molecule to a target adenine or cytosine residue in DNA within the site selectively recognized by the enzyme. Attachment of a fluorophore via a flexible linker to certain positions of the adenosyl moiety makes sure the dye does not interfere with cofactor binding. Labeling of DNA is carried out by using AdoMet-dependent MTases, and the adenosyl moiety serves as the molecular anchor for cofactor binding. This approach is mimicked by "pro-aziridine" cofactors, such as N-mustard analogs of AdoMet (Zvag et al., (2006) J. Am. Chem. Soc. 128, 2760-2761). These compounds undergo methyltransferase-directed coupling of the whole cofactor molecule to a target adenine or cytosine residue in the DNA, in a process thought to occur via transient formation and opening of an aziridine ring. These analogs contain the adenosyl moiety and may contain the aminoacid side chain as well, to serve as achors for cofactor binding to the enzyme.

Another class of AdoMet analogs have exchanged the methyl group with a group capable of facilitation the enzyme mediated transfer from the activated sulfonium center, a process otherwise severely hampered by steric hindrance for groups larger than methyl. Such cofactors are named doubly-activated AdoMet analogs since they bear an activating double or triple bond in beta-position to the transferable carbon unit (Dalhoff et al., (2006) Nat. Chem. Biol. 2, 31-32). Here, the remainder of the molecule (Adenosine base, amino acid, pentose sugar) serve as molecular anchors for cofactor binding to the enzyme and only part of its molecule (the activated side chain) is transferred onto a target nucleobase in the enzyme recognition site.

However, the labeling strategies that exploit the above cofactor analogs bear the following shortcomings:
a) The cofactor analogs are chemically unstable and thus exhibit short half-lifes under physiological conditions (V. Goyvaerts, Chem. Commun., (2020)). This may limit the effective use of the cofactors, both in stability during the assays as well as in storage and shipping. They may require storage in special buffers at low temperature (-20° C to -80° C).
b) The labeling reaction is not very general, and depends highly on enzyme permissiveness. Certain enzymes are capable of transferring a broad variety of functionalities (e.g. M.Taql transfers many different non-natural substrates to DNA), whereas the process is inefficient with most other wild type methyltransferases due to increased steric bulk of the transferable side chain. One solution to this problem is engineering of the cofactor binding pocket of the methyltransferase by site-directed mutagenesis (Lukinavicius et al., (2007) J. Am. Chem. Soc. 129, 2758-2759). However, it is not clear if this approach will be successful for other enzymes, since successful engineering examples come only from a single class methyltransferase enzymes. This requires cumbersome studies to optimize enzyme-cofactor match and activity, and may limit access to other DNA recognition sequences and thus hamper the applicability of the method.
c) All previously known labeling reactions transfer functionality to a specific nucleobase within a the recognition sequence of the DNA. However, this imparts that transfer is blocked by existing epigenetic modifications of this nucleobase, such as in the case of pre-existing DNA methylation or hydroxymethylation. In itself, this blocking effect can be used by certain technologies to gain information on the epigenetic nature of this one nucleobase (e.g. US2016355542A1 UNIVERSAL METHYLATION PROFILING METHODS). However, since no functionality is transferred on such sites, this poses difficulties for other applications such as genomic mapping, where presence of the full recognition sequence is inferred from the transfer to a single nucleobase within the recognition sequence. This is then an error in the signal, and leads to low performance.

Therefore, new methods in DNA handling and labeling that further leverage the natural sequence specific DNA recognition of methyltransferase enzymes but rely on more stable compounds and are not sensitive to existing nucleobase modifications in the recognition sequence will help to overcome these constraints.

### Summary of Invention

The present invention relates to and includes methods and compositions for enzyme guided labeling of biomolecules. Such labeling result from the application of agents that interact with a protein capable of recognizing specific sites of biomolecules and subsequently reacting covalently with the biomolecule at or near the specific site.

One aspect of the present disclosure relates to a compounds represented by formula (I): wherein 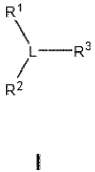
R¹ is a ligand for proteins or other macromolecules capable of selectively recognizing a biomolecule or sites on a biomolecule
R² is a reactive group capable of binding to the biomolecule
R³ is a functional group which can be used to generate a signal
L is a linker covalently attaching R¹, R², R³ to each other in a linear or branched manner and in no specific order

In a further embodiment, the inventions relates to a compounds represented by formula (I):
wherein
R¹ is a selective binder of S-adenosyl-L-methionine-dependent methyltransferase enzymes

Such compounds, in the presence of a methyltransferase, were found to be selectively coupled to a locus on a biomolecule that is near or in the natural target of the directing methyltransferase, but therefore not necessarily coupled to the actual target of the methyltransferase. For DNA methyltransferases, this coupling target is a single nucleobase within the recognition site of the enzyme.

In one aspect the present invention also relates to a complex of a compound as described above and a methyltransferase capable of using S-adenosyl-L-methionine as a cofactor. The present invention furthermore relates to a kit comprising a compound (I) as described above, or a complex as described above, packed in a container.

The present invention furthermore relates to a pharmaceutical or diagnostic composition comprising a compound as described above or a complex as described above.

The present invention also relates to a method for the preparation of a modified target molecule, the method comprising the incubation of the target molecule with a compound (I) as described above in the presence of a methyltransferase which is capable of binding the compound (I) and under conditions which allow for the transfer of part of the compounds onto the target molecule. This may for example require incubating a methyltranseferase enzyme with a compound as described above in a suitable aqueous buffered solution for the appropriate time, followed by a purification of the substrate, which usually comprises proteinase treatment of the sample followed by purification through chromatography or precipitation.

The present invention also relates to a method for detecting structural information in a biomolecule, comprising:
(a) contacting a biomolecule with an S-adenosyl-L-methionine-dependent methyltransferase in the presence of a compound (I) as described above; and
(b) detecting the labels wherein the pattern of labels on the biomolecule is indicative of the biomolecule structure and/or identity.

Particular and preferred aspects of the invention are set out in the accompanying independent and dependent claims. Features from the dependent claims may be combined with features of the independent claims and with features of other dependent claims as appropriate and not merely as explicitly set out in the claims.

In some embodiments, the present invention can be used for the analysis of DNA

These and other aspects of the invention will be apparent from and elucidated with reference to the embodiment(s) described hereinafter.

### Description of drawings

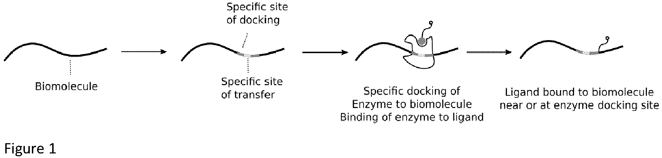

Drawing 1 is a schematic depiction of one embodiment of the invention

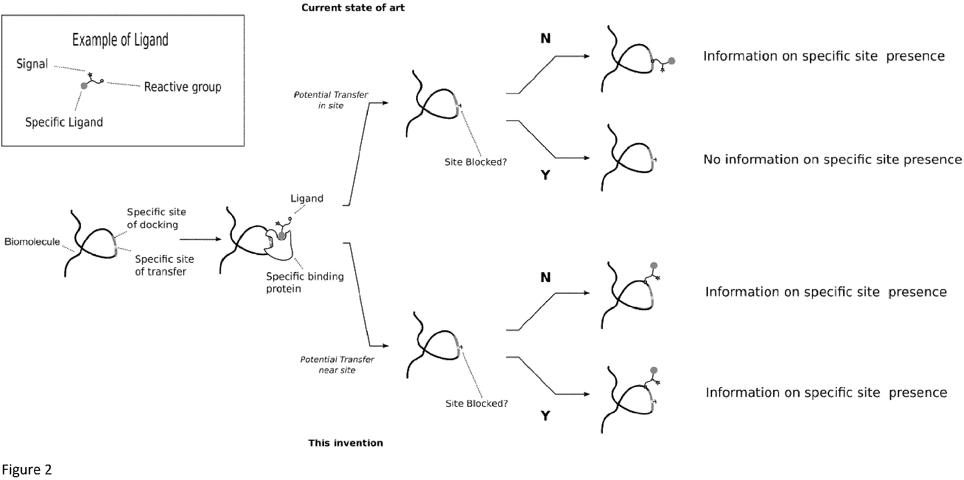

Drawing 2 is a schematic depiction of one embodiment of the invention

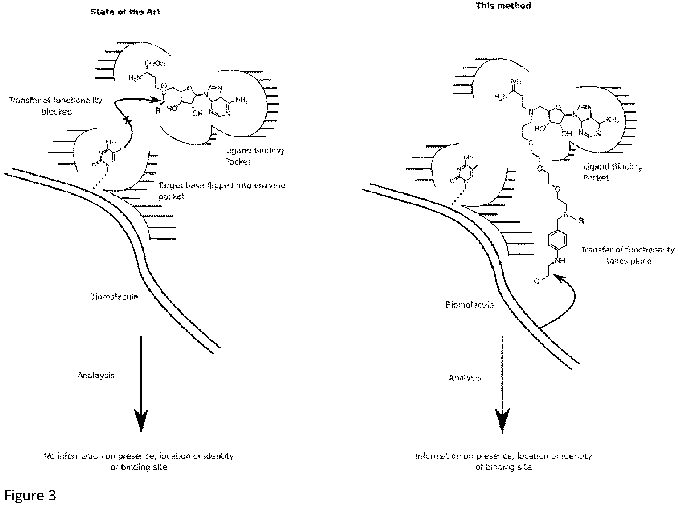

Drawing 3 is a schematic depiction of the mechanisms behind a notable advantage of the methods of the invention over the state of the art

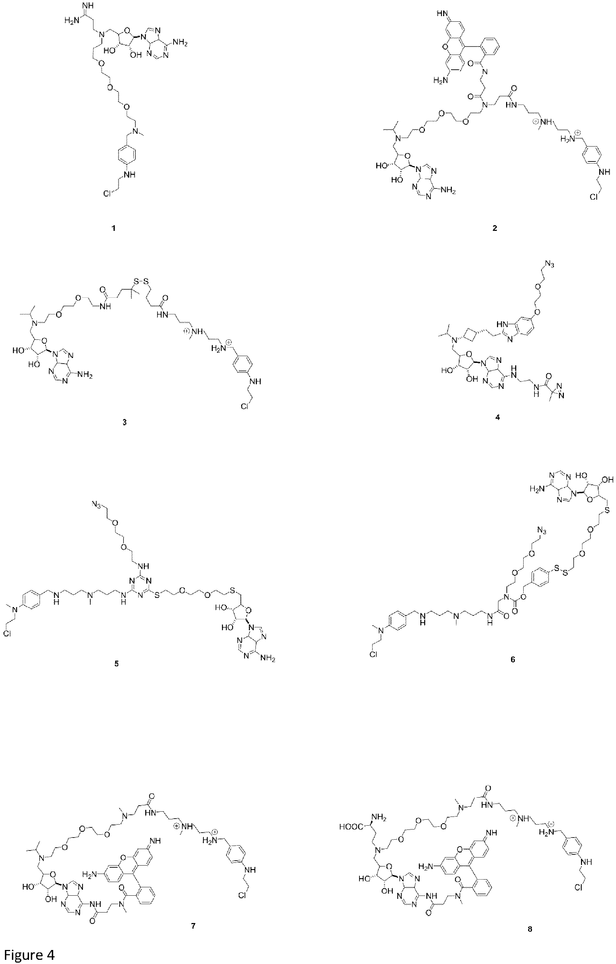

Drawing 4 are various examples of enzyme ligands as described in the invention

### Definitions

The following terms and related definitions are used in the present text.

"Subject" is used herein to mean any living being, human or animal. Nevertheless, the here disclosed method can be used for plants as well. As it is obvious for those skilled in the art, that subject in the context of this patent should mean any living body exposed to a viral infection.

"Sample" is used herein to mean first, any substance taken from a subject and undergoing a diagnosis based on the disclosed method. Secondly, our method applies equally well to any material like textiles, plastics, air filters, but not limited hereto. In summary, sample is used here for designating any living material and any solid or liquid or gaseous material where polynucleotides may be present. A sample taken from a subject may contain biological material such as saliva, mucus, cheek swabs, nasal swabs, blood, fecal matter, urine, or substances from breather masks, dust recovered from air filters, surface swabs but not limited hereto. For efficient early detection in populations these samples may be pooled

"Stochastic" random probability distribution or pattern that may be analysed statistically but may not be predicted precisely

"Stretching" is used herein to mean depositing a DNA molecule onto a surface so that all vectors that point form a nucleotide n to the neighboring nucleotide n+1 or n-1 have a positive projection onto the vector from the first nucleotide to the last one. By these kind of approach the base pair distance is increased and acts like an additional magnification for an optical reading.. Effectively this means that a DNA forms linear object, where the DNA strand along the stretching may have up to several micrometer, but in the lateral, perpendicular to the stretching direction is limited to several nanometers.

"Optical read out" is used herein to mean: a method that uses light signals to glean a specific information allowing the identification with high accuracy of viral species. Such signal or optical intensity profiles are put into relation with the genetic codes known and downloaded from a databank. A matching algorithm can relate with high accuracy the measured signal to an priori known RNA or DNA based information, allowing to assign the measured signal to a known genetic information.

"Biorthogonal" is used herein to mean: chemical reactions that can be used in biological systems, coupling one reactive group specifically with another reactive group: without side reactions; in neutral, aqueous solution; and under additional conditions that are compatible with the biological system. Selective reaction between bioorthogonal binding partners can minimize side reactions with other binding agents, biological compounds, or other non-complementary bioorthogonal binding agents or non-complementary bioorthogonal functional groups. Bioorthogonal functional groups of bioorthogonal binding agents include, but are not limited to, an azide and alkyne for formation of a triazole via Click-chemistry reactions, trans-cyclooctene (TCO) and tetrazine (Tz) (e.g., 1,2,4,5-tetrazine), and others. The binding agents useful in the present disclosure may have a high reactivity with the corresponding binding agent so that the reaction is rapid.

The term "complementary" as used herein refers to the hybridization or base pairing between nucleotides or nucleic acids, such as, for instance, between the two strands of a double stranded DNA molecule or between an oligonucleotide primer and a primer binding site on a single stranded nucleic acid to be sequenced or amplified. Complementary nucleotides are, generally, A and T (or A and U), or C and G. Two single stranded RNA or DNA molecules are said to be complementary when the nucleotides of one strand, optimally aligned and compared and with appropriate nucleotide insertions or deletions, pair with at least about 80% of the nucleotides of the other strand, usually at least about 90% to 95%, and more preferably from about 98 to 100%. Alternatively, complementarity exists when an RNA or DNA strand will hybridize under selective hybridization conditions to its complement. Typically, selective hybridization will occur when there is at least about 65% complementary over a stretch of at least 14 to 25 nucleotides, preferably at least about 75%, more preferably at least about 90% complementary.

The term "affinity ligand" refers to a molecule having an ability to bind to a specific molecule by specific affinity, and in the present invention, the term refers to molecules capable of selectively binding to a protein or aptamer. Note that the affinity ligand may be simply referred to as "ligand".

The terms "binding" and "binding agent" refers to a chemical or biological agent that specifically binds to a target (e.g., a targeted biomolecule), thereby forming a stable association between the targeting agent and the specific target. By "stably associated" or "stable association" is meant that a moiety is bound to or otherwise associated with another moiety or structure under standard physiological conditions. Bonds may include covalent bonds and non-covalent interactions, such as, but not limited to, ionic bonds, hydrophobic interactions, hydrogen bonds, van der Waals forces (e.g., London dispersion forces), dipole-dipole interactions, and the like. A targeting agent may be a member of a specific binding pair, such as, but not limited to: a member of a receptor/ligand pair; a ligand-binding portion of a receptor; a member of an antibody/antigen pair; an antigen-binding fragment of an antibody; a hapten; a member of a lectin/carbohydrate pair; a member of an enzyme/substrate pair; biotin/avidin

The term "contacting" or "contact" refers to the process of bringing into contact at least two distinct species such that they can interact with each other, such as in a non-covalent or covalent binding interaction or binding reaction. It should be appreciated, however, the resulting complex or reaction product can be produced directly from an interaction or a reaction between the added reagents or from an intermediate from one or more of the added reagents or moieties, which can be produced in the contacting mixture.

The term "linker", "linked" or "linking" refers to a chemical moiety that attaches two moieties together, such as a compound of the present disclosure to a biological material that targets a specific type of cell, such as a cancer cell, other type of diseased cell, or a normal cell type. The linking can be via covalent bonds, ionic bonds, hydrophobic interactions, hydrogen bonds, van der Waals forces (e.g., London dispersion forces), dipole-dipole interactions, and the like. The linking can be direct linkage between to the two moieties being linked, or indirectly, such as via a linker. Linkers useful in embodiments of the present disclosure include linkers having 30 carbon atoms or less in length. In some embodiments, the linkers are 1-15 carbon atoms in length, such as 1-12 carbon atoms, or 1-10 carbon atoms, or 5-10 carbon atoms in length. Representative linkers can have 1 to 100 linking atoms, and can include, but are not limited to, ethylene-oxy groups, amines, esters, amides, carbamates, carbonates, and ketone functional groups. For example, linkers may have from 1 to 50 linking atoms, or from 1-30 linking atoms. Other types of bonds may also be used in embodiments of the present disclosure.

The term "binding agent" refers to an agent having a functional group capable of forming a covalent bond to a complementary functional group of another binding agent in a biological environment. Binding between binding agents in a biological environment may also be referred to as bioconjugation. Representative binding agents include, but are not limited to, an amine and an activated ester, an amine and an isocyanate, an amine and an isothiocyanate, thiols for formation of disulfides, an aldehyde and amine for enamine formation, an azide for formation of an amide via a Staudinger ligation. Binding agents also include bioorthogonal binding agents, which are binding agents having bioorthogonal functional groups.

"Nucleic acids" or "polynucleotides" of the invention include, but are not limited to, ribonucleic acids (RNAs), deoxyribonucleic acids (DNAs), threose nucleic acids (TNAs), glycol nucleic acids (GNAs), peptide nucleic acids (PNAs), locked nucleic acids (LNAs, including LNA having a β-D-ribo configuration, α-LNA having an α-L-ribo configuration (a diastereomer of LNA), 2'-amino-LNA having a 2'-amino functionalization, and 2'-amino-α-LNA having a 2'-amino functionalization), ethylene nucleic acids (ENA), cyclohexenyl nucleic acids (CeNA) or hybrids or combinations thereof.

By the phrase "nucleic acid extraction reagent" is meant any reagent (e.g., solution) that can be used to obtain a nucleic acid (e.g., DNA) from biological materials such as cells, tissues, bodily fluids, microorganisms, etc. An extraction reagent can be, for example, a solution containing one or more of: a detergent to disrupt cell and nuclear membranes, a proteolytic enzyme(s) to degrade proteins, an agent to inhibit nuclease activity, a buffering compound to maintain neutral pH, and chaotropic salts to facilitate disaggregation of molecular complexes.

"Reactive group" refers to a chemical moiety capable of reacting with a partner chemical moiety to form a covalent linkage or non-covalent linkage. A moiety may be considered a reactive group based on its high reactivity with a single partner-moiety, a set of partner-moieties, or based on its reactivity with many partners.

"DNA Mapping" refers to a process where sequence specific markers are introduced to a polynucleotide, and where the distance information between these markers yields information on the genetic makeup of the polynucleotide. DNA mapping may refer to all polynucleotides in a sample, including but not limited to genomic DNA, plasmid DNA, mRNA, tRNA and genomic RNA.

### Detailed description of invention

The present invention will be described with respect to particular embodiments and with reference to certain drawings but the invention is not limited thereto but only by the claims. Furthermore, the terms first, second and the like in the description and in the claims, are used for distinguishing between similar elements and not necessarily for describing a sequence, either temporally, spatially, in ranking or in any other manner. It is to be understood that the terms so used are interchangeable under appropriate circumstances and that the embodiments of the invention described herein are capable of operation in other sequences than described or illustrated herein.

It is to be noticed that the term "comprising", used in the claims, should not be interpreted as being restricted to the means listed thereafter; it does not exclude other elements or steps. It is thus to be interpreted as specifying the presence of the stated features, integers, steps or components as referred to, but does not preclude the presence or addition of one or more other features, integers, steps or components, or groups thereof. Thus, the scope of the expression "a device comprising means A and B" should not be limited to devices consisting only of components A and B. It means that with respect to the present invention, the only relevant components of the device are A and B.

Reference throughout this specification to "one embodiment" or "an embodiment" means that a particular feature, structure or characteristic described in connection with the embodiment is included in at least one embodiment of the present invention. Thus, appearances of the phrases "in one embodiment" or "in an embodiment" in various places throughout this specification are not necessarily all referring to the same embodiment, but may. Furthermore, the particular features, structures or characteristics may be combined in any suitable manner, as would be apparent to one of ordinary skill in the art from this disclosure, in one or more embodiments.

Similarly it should be appreciated that in the description of exemplary embodiments of the invention, various features of the invention are sometimes grouped together in a single embodiment, figure, or description thereof for the purpose of streamlining the disclosure and aiding in the understanding of one or more of the various inventive aspects. This method of disclosure, however, is not to be interpreted as reflecting an intention that the claimed invention requires more features than are expressly recited in each claim. Rather, as the following claims reflect, inventive aspects lie in less than all features of a single foregoing disclosed embodiment. Thus, the claims following the detailed description are hereby expressly incorporated into this detailed description, with each claim standing on its own as a separate embodiment of this invention.

In the description provided herein, numerous specific details are set forth. However, it is understood that embodiments of the invention may be practiced without these specific details. In other instances, well-known methods, structures and techniques have not been shown in detail in order not to obscure an understanding of this description.

One aspect of the present disclosure relates to a compound represented by formula (I): 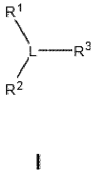 wherein
R¹ is a ligand of S-adenosyl-L-methionine-dependent methyltransferase enzymes
R² is a reactive group comprising at least one member of reactive groups capable of binding to DNA, such as Platinum compounds, azides, diazirines, mustards, carbene sources, nitrene sources, alkynes, minor groove binders. This binding can be covalent, non-covalent and permanent or non-permanent.
R³ is a functional group comprising at least one member selected from fluorophores, fluorescence quenchers, affinity tags, crosslinking agents, nucleic acid cleaving reagents, spin labels, heavy atoms or heavy atom clusters suitable for phasing of X-ray diffraction data, radioactive or stable rare isotopes, chromophores, proteins, peptides or amino acids which may optionally be modified, nucleotides, nucleosides, nucleic acids which may optionally be modified, carbohydrates, lipids, transfection reagents, intercalating agents, nanoparticles, beads, and a functional group being selected from an amino group, a thiol group, a 1 ,2-diol group, a hydrazino group, a hydroxyamino group, a haloacetamide group, a maleimide group, an aldehyde group, a ketone group, an 1,2-aminothiol group, an azido group, an alkyne group, a 1 ,3-diene function, a dienophilic function (e.g. activated carbon-carbon double bond), an arylhalide group, a terminal alkyne group, an arylboronic acid group, a terminal haloalkyne group, a terminal silylalkyne group and a protected amino, thiol, 1 ,2-diol, hydrazino, hydroxyamino, aldehyde, ketone and 1,2-aminothiol group.
L is a linker covalently attaching R¹, R², R³ to each other in a linear or branched manner and in no specific order.

In one embodiment, the selective binder of S-adenosyl-L-methionine-dependent methyltransferase enzymes is represented by formula (II) Wherein 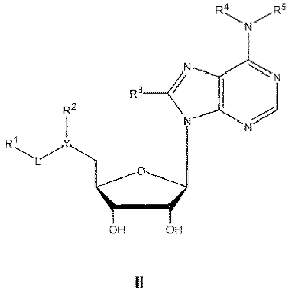
R¹ is a reactive group comprising at least one member of reactive groups capable of binding to DNA, such as metal complexes, azides, diazirines, mustards, carbene sources, nitrene sources, alkynes, minor groove binders, major groove binders, DNA intercalators.
R² is independently selected from H, CH2COOH, CH2(NH2)COOH, CH2CH2(NH2)COOH,CH2 CH2COOH, CH2CH2(NH2)(CN4H)
R³ is H or CH₃
R⁴ is H or CH₃
R⁵ is a functional group comprising at least one member selected from fluorophores, fluorescence quenchers, affinity tags, crosslinking agents, nucleic acid cleaving reagents, spin labels, heavy atoms or heavy atom clusters suitable for phasing of X-ray diffraction data, radioactive or stable rare isotopes, chromophores, proteins, peptides or amino acids which may optionally be modified, nucleotides, nucleosides, nucleic acids which may optionally be modified, carbohydrates, lipids, transfection reagents, intercalating agents, nanoparticles, beads, and a functional group being selected from an amino group, a thiol group, a 1 ,2-diol group, a hydrazino group, a hydroxyamino group, a haloacetamide group, a maleimide group, an aldehyde group, a ketone group, an 1,2-aminothiol group, an azido group, an alkyne group, a 1 ,3-diene function, a dienophilic function (e.g. activated carbon-carbon double bond), an arylhalide group, a terminal alkyne group, an arylboronic acid group, a terminal haloalkyne group, a terminal silylalkyne group and a protected amino, thiol, 1 ,2-diol, hydrazino, hydroxyamino, aldehyde, ketone and 1,2-aminothiol group.
L is a linker.
Y is N or CH

In a further aspect of the present invention, the linker arm L is a chain of atoms of any length that may be comprised of carbon, nitrogen, oxygen, sulfur in any combination and any other possible atoms. The connecting chain can be saturated, unsaturated or can contain aromatic rings and the linking chain can be flexible or rigid. The connecting chain can further comprise any of the rigid units previously disclosed in U.S. Patent Publication 2005/0137388. In this aspect of the invention, examples of reactive groups can include but not be limited to active esters, groups capable of forming a carbon-carbon bonds and groups capable of forming bonds with O, N or S. Examples of such groups can include but not be limited to isothiocyanate, isocyanate, monochlorotriazine, dichlorotriazine, mono- or di-halogen substituted pyridine, mono- or di-halogen substituted diazine, maleimide, aziridine, sulfonyl halogen substituted diazine, maleimide, aziridine, sulfonyl halide, acid halide, hydroxysuccinimide ester, hydroxysulfosuccinimide ester, imido ester, hydrazine, azidonitrophenyl, azide, 3-(2-pyridyl dithio)-proprionamide, glyoxal, aldehyde, carbon-carbon double bonds, mercury salts, and any group capable of reacting with carbon-carbon double bonds, amines, hydroxyl groups, sulfhydryl groups and halogens. The reactive groups may also participate in formation of a coordinate bond when R comprises a ligand or a metal. A reactive group R can be attached to the oligomeric or polymeric moiety through a linker arm L as described above or if desired it may be attached directly without the use of a linker arm. It is a further aspect of this invention that the reactive group can be chemically linked to the novel labeling reagent at a terminus, a side chain or an internal site of the oligomeric or polymeric moiety. Furthermore, the novel polymeric composition described may also contain additional alkyl, aryl and/or polar or charged groups on the backbone, linking arm or the dyes or labels. The polar or charged groups may include but are not limited to halogen, substituted or unsubstituted alkyl or aryl groups, saturated or unsaturated alkyl groups, alkoxy, phenoxy, amino, amido, and carboxyl groups, polar groups such as nitrates, sulfonates, sulfhydryl groups, nitrites, carboxylic acids, phosphates or any other such group or substituent.

In a further aspect of the present invention, the Linker arm L may assist DNA binding to ensure favourable positioning of the reactive group R¹. This can for example be achieved through the incorporation of groups that are positively charged or become positively charged upon dissolution in the medium, facilitating binding to the negatively charged phosphate backbone of DNA. Alternatively, the linker contains a minor or major groove binding moiety to position the reactive group R1 in the vicinity of the DNA. Examples thereof are netropsin, distamycin, Hoechst 33258, pentamidine, DAPI or oligomers of nitrogen containing heterocycles.

In a further aspect of the present invention, the Linker arm L may be cleavable upon a trigger. This trigger can be chemical (e.g. pH shift, reduction), physical (e.g. Thermal, light induced) or enzymatic (e.g. proteolytic).

The binding of the reactive group to the biomolecule can be initiated by a trigger. This trigger can be chemical (e.g. pH shift, reduction), physical (e.g. Thermal, light induced) or enzymatic (e.g. proteolytic).

In stark difference to previously described methyltransferase based DNA labeling, the enzyme plays no active role in the actual covalent binding of the label to the DNA, but serves to direct this binding event to a specific location in or near its recognition sequence.

As a unique and non-obvious benefit of the method, we show that labeling outside the actual enzymatic transfer site can still provide identity and structure information

An important aspect if this invention is that the independency of transfer of a group from the natural cofactor to the biomolecule target within its defined labeling site allows the invention to extend into a broad range of enzyme binding structural motifs. For methyltransferases, a non-limiting description of such structural motifs are described in Zhao et al, Epigenetic Targets and Their Inhibitors in Cancer Therapy, Current Topics in Medicinal Chemistry, 19 (28), 2018; Copeland et al., Protein methyltransferase inhibitors as precision cancer therapeutics: a decade of discovery, Phil. Trans. R. Soc. B3732017008020170080, 2018.

In a specific embodiment, the label on the DNA is a reactive group and after enzyme directed DNA labeling, this reactive group is reacted with a label in a chemical reaction.

In a preferred embodiment, this reaction is biorthogonal

In a preferred embodiment, this reaction is selected from the Staudinger ligation, the azide-cyclooctyne cycloaddition, azide-alkyne cycloaddition, thiol-ene reaction and the inverseelectron- demand Diels- Alder reaction

In the labeling methods, DNA compositions, and kits of some embodiments disclosed herein, a enzyme directed chemical labeling process is used to label selected target sequences on DNA.

The target biomolecule can be polynucleotides, RNA, DNA, peptides, proteins, lipids and small molecules.

In some embodiments, the method further comprises detecting a relative distance between the labels on the linearized DNA, thus providing a barcode of a portion of the genomic DNA. This information can be used in genomic analysis. In some embodiments, this distance can be detected by linearizing the labeled DNA in a fluidic channel, in which the DNA remains intact upon said linearization. In some embodiments, the distance can be detected by linearizing the labeled DNA on a surface. In some embodiments, the distance can be detected by passing the labeled DNA through a nanopore.

In some embodiments, the method is used for the analysis of polynucleotides. In some embodiments, the polynucleotide is genomic DNA. In some embodiments, the analysis of genomic DNA can be used for species identification, where these species are single species, or mixtures of species, as to identify the presence of species or the composition of the mixture of species.

In some embodiments, the method is used for the analysis of biomolecules through selective isolation or enrichment of subset of biomolecules. Such isolation can be effected through the selective reaction with a functional group placed on the biomolecule through the methods of the invention.

It is further contemplated that the labeling reaction can be selected to overcome some of the current limitations of the state of the art. For example, DNA labeling by the combination of a methyl transferase and a non-natural cofactor is blocked by existing epigenetic modification. By selecting a chemical method of labeling that is not hindered by the underlying epigenetic modification, for example reacting with the entire nucleobase, labeling can still take place at locations otherwise blocked.

In another embodiment, the genomic DNA is contacted with multiple methyltransferases, each agent having a different target sequence in the genomic DNA, wherein each target nucleic acid sequence is detected via the same or different label, thus providing a barcode of a portion of the genomic DNA. In some embodiments, the method further comprises labeling the DNA by an additional chemistry, for example direct enzymatic labeling using an enzyme and optionally further including a stain in addition to the enzymatic labeling, or nicking followed by nick labeling and repair to produce a DNA with two or more different specificity motifs with different labels (e.g., different colors).

The methyltransferase used to guide the labeling can be selected in light of its sequence specificity. Non-limiting exemplary Methyltransferase enzymes are discussed in, for example, U.S. Pat. No. 8,008,007, published Aug. 30, 2011, and US patent application No. US2021010074A1 both of which are hereby expressly incorporated by reference in its entirety. Noteworthy, it should also be useful to access methyltransferase guided labeling of RNA or (poly)peptides or other biomolecules within or near the enzymatic binding site. Additional or alternate methyltransferase enzymes are also contemplated herein.

In contrast with non-natural methyltransferase cofactors and their methyltransferase enzyme combinations the wild-type enzyme may be used without modification. However, further modifications may be made to the methyltransferase to optimize functionality (e.g. to increase the binding efficiency) in individual cases.

Importantly and unlike other methyltransferase based DNA labeling schemes, the teachings of the present invention allows methyltransferase guided labeling in a methylationindependent manner. For other methyltransferase labeling schemes, transfer of groups to nucluobases is hindered by existing epigenetic modifications of the target nucleobases, such as methylation, hydroxymethylation, carbonylation or carboxylation. Since the methods described do not necessarily transfer to that nucleobase, they do not suffer from the impact the existing epigenetic marks will have on the analysis.

It is contemplated that these methods can extend into DNA labeling with restriction and nicking enzymes. A typical features of these enzymes are their obligate sequence specificity but also a requirement for metal cations (mostly 2+) during catalysis, with few exceptions. By combining these enzymes with a specific ligand and with no metal cations present, the use of such enzymes can be extended into novel approaches of DNA labeling.

It is contemplated that these methods extend to other enzymes and proteins that bind to specific sites on a polynucleotide or to specific states of a polynucleotide. Examples of such enzymes and proteins that bind to polynucleotides in a sequence specific manner outside methyltransferases are transcription factors or proteins containing DNA binding domains such as Helix-turn-helix, Zinc finger, Leucine zipper, Winged helix, Winged helix-turn-helix, Helix-loop-helix, HMG-box, Wor3 domain, OB-fold domain, Immunoglobulin fold, B3 domain or TAL effector. A specific case of DNA binding proteins is proteins guided by RNA. For example Cas9 can be used as a customizable RNA-guided DNA-binding platform, and when combined with ligands as disclosed in this invention, be able to effect targeted DNA modification.

An additional example may be enzymes binding to accessible chromatin, transfer functionality to said chromatin and enable specific analysis of chromatin accessibility and its uses in genetic analysis.

Further embodiments of the inventions may substitute the enzyme for synthetic macromolecules capable of recognizing specific docking positions on the biomolecule. An example of such macromolecules can be an aptamer.

It is further contemplated that the ligand may bind to the enzyme or protein with no or only partial overlap to the standard binding pocket of the natural cofactor. This has the significant and non-obvious advantage of the methods described herein that remaining natural enzymatic substrate does not hinder the reactions course, as is often the case when non-natural cofactor substrates are used. Advantageously, interactions between the ligand and the nature over their binding may actually improve performance and specificity (Shapira et al, Chemical inhibition of Protein Methyltransferases, Cell Chemical Biology, 2016, Volume 23, issue 9, 1067).

For the methods and compounds described in this invention, it is envisioned that the order of addition of all components can be varied. The DNA binder and its ligand can be added sequentially and in varying order to the polynucleotide to effect labeling. Alternatively, the DNA binder and its ligand can be combined to form a stable complex that is capable of binding to the sequence target and then effect the labeling. Such a preformation of an active labeling reagent is non-obvious and solves a current problem, where for example in the case of current methods with methyltransferase enzymes and non-natural cofactors, preincubation with the enzyme is detrimental to cofactor stability and integrity, as the cofactor reacts with the proteine. As such, the methods and compounds of this invention hold significant benefits for formulation and flexibility of use.

Furthermore, the methods have the advantage that transfer-efficiency of the enzyme is no longer governed by the ability of the enzyme to catalyze the transfer of a non-natural substrate, but only require efficient binding to the enzyme or protein. Unlike for the natural cofactor SAM, where transfer is catalytic and fast, large equivalents of non-natural cofactor are required to reach efficient transfer. Moreover, for GC transferases, very few non-natural cofactors exist that transfer efficiently, and enzyme mutations are required to accommodate for the non-natural enzyme. In the methods described, this is not the case, and transfer can be effected by vastly more methyltransferases, making the method much more flexible.

In any such method described above, (a) the DNA labeling reactions may be performed in the same reaction vessel, or (b) DNA from the sample may be divided into aliquots and added to different reaction vessels, and wherein DNA labeling reactions are performed in each reaction vessel.

In the kits of the invention at least one labelling entity may be a fluorophore or a molecule to which a fluorophore may be attached.

Any of the kits of the invention may further comprise at least one affinity chromatography column.

Any of the kits of the invention may further comprise at least one buffer.

Any of the kits of the invention may further comprise one or more other enzymes for performing reactions required in methods of the invention. The enzymes may comprise additionaly comprise a DNA nickase or a DNA polymerase.

Labels that are optically detectable are particularly useful. Examples include chromophores, luminophores and fluorophores. Fluorophores are particularly useful and include, for example, fluorescent nanocrystals; quantum dots, fluorescein, rhodamine, tetramethylrhodamine, eosin, erythrosin, coumarin, methyl-coumarins, pyrene, Malachite green, Cy3, Cy5, stilbene, Lucifer Yellow, Cascade Blue, Texas Red, Alexa dyes, SETA dyes, Atto dyes, phycoerythin, bodipy, and analogs thereof. Useful optical probes are described in Haugland, Molecular Probes Handbook, (Eugene, Oreg.) 6th Edition; The Synthegen catalog (Houston, Tex.), Lakowicz, Principles of Fluorescence Spectroscopy, 2nd Ed., Plenum Press New York (1999), or WO 98/59066; WO 91/06678 or US Pat. Appl. Publ. No. 2010/0092957 A1, each of which is incorporated herein by reference. Optical labels provide an advantage of rapid, relatively non-invasive detection thereby allowing real time monitoring of a cyclic reaction.

Other labels, some of which are non-optical labels, can be used in various applications of the methods and compositions set forth herein. Examples include, without limitation, an isotopic label such as a naturally non-abundant radioactive or heavy isotope; magnetic substance; electron-rich material such as a metal; electrochemiluminescent label such as Ru(bpy); or moiety that can be detected based on a nuclear magnetic, paramagnetic, electrical, charge to mass, or thermal characteristic. Labels can also include magnetic particles or optically encoded nanoparticles. Such labels can be detected using appropriate methods known to those skilled in the art. For example, a charged label can be detected using an electrical detector such as those used in commercially available sequencing systems from Ion Torrent (Guilford, Conn., a Life Technologies subsidiary) or detection systems described in US Pat. App. Publ. Nos. 2009/0026082 A1; 2009/0127589 A1; 2010/0137143 A1; and 2010/0282617 A1, each of which is incorporated herein by reference. It will be understood that for some applications a nucleotide analog need not have a label.

Another type of label that can be useful is a secondary label that is indirectly detected, for example, via interaction with a primary label, binding to a receptor or conversion to a detectable product by an enzyme catalyst or other substance. An example secondary label is a ligand such as biotin or analogs thereof that can be detected via binding to a receptor such as avidin, streptavidin or analogs thereof. Other useful ligands are epitopes that can bind to receptors such as antibodies or active fragments thereof, and carbohydrates that can bind to receptors such as lectins. The receptors can be labeled, for example, with an optical label, to allow them to be detected. In particular applications, the ligand can be attached to a nucleotide analog in a way that reduces or prevents affinity to a receptor. Release of the ligand can then be detected based on affinity of the ligand for its respective receptor when detached from the nucleotide analog. The ligand can further be attached to a blocking moiety or may itself function as a blocking moiety, as set forth above more generally for label moieties. Thus, removal of the ligand from a nucleotide analog can function to deblock the nucleotide analog and to provide a detectable event.

### Examples

### Example 1

Lambda phage DNA is incubated with in the presence of M.Taql methyltransferase and compound 5 (Image 3) at 55°C for 60 minutes. Rhodamine B-Cyclooctyne is added (10 equivalents) and the reaction is shaken overnight at room temperature. The labeled DNA is purified by phenol-chloroform extraction and ethanol precipitation followed by genomic mapping analysis to indicate sequence specific labeling near the M.Taql recognition site.

## Claims

1. A method for covalent modification of biomolecules, comprising contacting a biomolecule with
i. A macromolecule capable of structure or site specific interaction with a biomolecule
ii. A ligand for the macromolecule, capable of forming a bond to the biomolecule
iii. Forming a bond between the ligand and the biomolecule near or at the site of interaction of the macromolecule
iv. Obtaining information about the biomolecule by addressing the covalently bound ligand

2. A method according to claim 1 where the macromolecule is an enzyme

3. A method according to claim 2 where the enzyme is a DNA methyltransferase enzyme

4. A method according to claim 3 where the ligand is a ligand for S-adenosyl-L- methionine (AdoMet) dependent methyltransferase enzymes

5. A method according to claim 4 where the biomolecule is DNA

6. A method according to claim 4 where the information is obtained through DNA sequencing

7. A method according to claim 5 where the DNA is analyzed through genomic mapping
